# EUROPÄISCHE PATENTANMELDUNG

(11) **EP 3 726 190 A1**
(43) Veröffentlichungstag der Anmeldung: **21.10.2020**
(21) Anmeldenummer: 20169920.4
(22) Anmeldetag: 16.04.2020
(51) Int. Cl.: G01J 5/00, G01J 5/02, G01J 5/04, G01J 5/08, G01J 5/52, A01K 29/00, A61B 5/00

(54) **VORRICHTUNG, BAUSATZ UND VERFAHREN ZUR BERÜHRUNGSLOSEN ERMITTLUNG DER KÖRPERTEMPERATUR EINES NUTZTIERES**

(30) Priorität: 18.04.2019 DE 102019110304
(71) Anmelder: Polten, Bernhard, 53347 Alfter (DE)
(72) Erfinder: Polten, Bernhard, 53347 Alfter (DE)
(74) Vertreter: Weickmann & Weickmann PartmbB

(57) **Zusammenfassung**

Die Erfindung betrifft eine Vorrichtung zur berührungslosen Ermittlung der Körpertemperatur eines Tieres bzw. Nutztieres (6), wobei die Vorrichtung aufweist: eine Tierversorgungseinrichtung (2) und/oder Tierpflegeeinrichtung, die für ein Tier bzw. Nutztier (6) selbstständig erreichbar ist; einen Temperatursensor (20) zur Messung der Oberflächen-Temperatur an mindestens einer Stelle des Körpers des Tieres bzw. Nutztieres (6), wobei der Temperatursensor (20) so an oder benachbart zu der Tierversorgungseinrichtung (2) und/oder Tierpflegeeinrichtung angeordnet ist oder angeordnet werden kann, dass der Temperatursensor (20) das die Einrichtung aufsuchende Nutztier (6) erfasst; eine Auswerteeinheit (30), die mit dem Temperatursensor (20) für einen Datenaustausch in Verbindung steht und die die vom Temperatursensor (20) gemessene Oberflächen-Temperatur erfasst, wobei die Auswerteeinheit (30) dazu eingerichtet ist, Temperaturdaten einem bestimmten Nutztier zuzuordnen und zu speichern; und eine Sende- und/oder Anzeigeeinheit (40), die mit der Auswerteeinheit (30) für einen Datenaustausch in Verbindung steht, wobei die Sende- und/oder Anzeigeeinheit (40) bevorzugt ausgebildet und/oder eingerichtet ist zum Anzeigen und/oder Versenden von Temperaturdaten und darauf basierenden Informationen bevorzugt in Bezug auf das bestimmte Nutztier (6). Die Erfindung betrifft ebenfalls einen entsprechenden Bausatz und ein entsprechendes Verfahren.

## Beschreibung

Die Erfindung betrifft eine Vorrichtung und ein Verfahren zur Ermittlung der Körpertemperatur eines Tieres z.B. Nutztieres.

Bei der Beurteilung des Wohlbefindens und des Gesundheitszustandes eines Tieres ist neben allgemeinen Symptomen einer der aussagekräftigsten Parameter die Körpertemperatur der Tiere.

Bei größeren Herden von Nutztieren ist die kontinuierliche Überwachung des Gesundheitszustandes der einzelnen Tiere eine Aufgabe, die große Sachkunde erfordert und mit erheblichem Arbeitsaufwand verbunden ist. Trotzdem ist es sowohl im Sinne einer artgerechten Tierhaltung als auch aus ökonomischen Erwägungen unbedingt notwendig, gesundheitliche Probleme frühzeitig zu erkennen, um dem Tier Leid zu ersparen (Früherkennung) und das Risiko eines Übergreifens auf andere Tiere (z.B. des Bestandes - z.B. Tierseuchenbekämpfung) sowie den Behandlungsbedarf zu minimieren.

Als Standardmethode, um die Körpertemperatur zu bestimmen, wird in der Praxis gewöhnlich die manuelle rektale Messung angewandt. Sie ist - besonders bei größeren Herden - mit einigen grundsätzlichen Problemen verbunden. Zunächst ist für die Messung geschultes Fachpersonal erforderlich. Die Messungen sollten nach Möglichkeit regelmäßig (bis mehrfach täglich) vorgenommen werden. Das verursacht einen entsprechenden zeitlichen und wirtschaftlichen Aufwand. Die ermittelten Werte und ggf. der Temperaturverlauf sollten für jedes Tier protokolliert werden. Damit kann später der behandelnde Tierarzt seine diagnostischen Schlüsse daraus ziehen.

Eine weitere in der Praxis angewandte Möglichkeit besteht bei bestimmten Tierarten darin, einen sog. Temperaturbolus einzusetzen. Dieser wird in den Magen des Tieres eingebracht und kann damit die beschriebene rektale Methode der Temperaturermittlung ablösen. Der Temperaturbolus ist ein Messgerät, das beispielsweise eine Kuh dauerhaft im Pansen trägt und das die Temperatur über Funk an eine oder mehrere stationäre Empfangsstationen überträgt. Damit ist es möglich, das oben beschriebene Vorgehen weitgehend zu automatisieren. Es ergeben sich allerdings einige andere schwerwiegende Probleme: Der Bolus muss veterinärmedizinisch implantiert werden; er ist nicht wiederverwendbar; die Temperaturumgebung des Bolus im Magen schwankt mit Futter- und Trinkwasseraufnahme, wobei gerade im Pansen die Fermentationswärme eine erhöhte Temperatur verursacht, d. h. eine Abweichung vom tatsächlichen Körpertemperaturwert; und während die Tiere trinken, wird die Temperatur weiterhin stark erniedrigt.

Mit Blick auf den Temperaturbolus ist zu sagen, dass die hier zu erkennenden Nachteile gravierend sind, insbesondere was das Einsetzen des Bolus in den Tierkörper anbelangt, aber auch die zwangsläufig auftretenden Fehlmessungen. Besonders letztere können die Auswertung einer Temperaturkurve erheblich erschweren, wenn keine zusätzlichen Informationen über die Aktivität des betreffenden Tieres vorliegen. Im schlimmsten Fall können sie sogar zu einer Fehldiagnose führen.

Eine weitere Möglichkeit zur Überwachung besteht darin, einen Temperatursensor zur Messung der Oberflächen-Temperatur an einer Stelle des Körpers des Nutztieres zu fixieren oder zu implantieren. Dies ergibt allerdings auch Schwierigkeiten, denn die Fixierung und/oder Implantierung selbst ist aufwändig, muss zur Vermeidung/Reduzierung von Beschädigungen/Fehlmessungen geeignet an einer Körperstelle auf die richtige Weise erfolgen und es kann ebenfalls Fehlmessungen geben, z. B. bei Verschmutzung der entsprechenden Stelle.

Zwar gab es auch immer wieder Anregungen, eine berührungslose Messung zu nutzen; diese konnten sich aber in der praktischen Anwendung gerade auch aufgrund von Umstellungsproblematiken nicht durchsetzen. Es ist vielmehr so, dass diese Anregungen in der Praxis quasi als Sackgasse verworfen wurden und nicht weiter verfolgt werden, denn es fehlte an für Landwirte unkompliziert nutzbaren und technisch einfachen sowie vor allem zuverlässigen Lösungen.

Für die rechtzeitige und zuverlässige Früherkennung gesundheitlicher Probleme einzelner Individuen in größeren Herden muss der Landwirt daher bislang bei in der Praxis gebräuchlichen Lösungen einen erheblichen Aufwand betreiben, wenn er eines der in der Praxis üblichen Standardverfahren zur Temperaturmessung verwendet. Außerdem ist bei vielen gebräuchlichen Lösungen dazu veterinärmedizinisch geschultes Fachpersonal notwendig.

Zur Überwindung dieser Nachteile wird erfindungsgemäß eine Vorrichtung nach Anspruch 1, ein Bausatz nach Anspruch 13 und/oder ein Verfahren nach Anspruch 14 vorgeschlagen. Vorteilhafte Ausgestaltungen sind Gegenstand der abhängigen Ansprüche.

Eine erfindungsgemäße Vorrichtung zur berührungslosen Ermittlung der Körpertemperatur eines Tieres insbesondere. Nutztieres umfasst:
- eine Tierversorgungseinrichtung und/oder Tierpflegeeinrichtung, die für ein Nutztier selbstständig erreichbar ist, bevorzugt eine Einrichtung, die regelmäßig von dem Nutztier selbsttätig aufgesucht wird, wie beispielsweise eine Futterstelle, eine Tränke, eine Melkstation oder ein ein Durchgang/Treibgang z.B. ins Außengelände und/oder/ zu einer Waage;
- einen Temperatursensor zur Messung der Oberflächen-Temperatur an mindestens einer Stelle des Körpers des Nutztieres, wobei der Temperatursensor so an oder benachbart zu der Tierversorgungseinrichtung und/oder Tierpflegeeinrichtung angeordnet ist oder angeordnet werden kann oder positionierbar ist, dass der Temperatursensor das die Einrichtung aufsuchende Nutztier erfasst;
- eine Auswerteeinheit, die mit dem Temperatursensor für einen Datenaustausch in Verbindung steht, insbesondere durch Fernkommunikation und/oder drahtlos (Sender und Empfänger), und die die vom Temperatursensor gemessene Oberflächen-Temperatur erfasst, wobei die Auswerteeinheit dazu eingerichtet ist, Temperaturdaten einem bestimmten Nutztier zuzuordnen und zu speichern, wobei die Auswerteeinheit bevorzugt als eine Einheit und/oder integral mit dem Temperatursensor ausgebildet ist; und
- eine Sende- und/oder Anzeigeeinheit, die mit der Auswerteeinheit für einen Datenaustausch in Verbindung steht, insbesondere durch Fernkommunikation, wobei die Sende-, Empfangs- und/oder Anzeigeeinheit bevorzugt ausgebildet und/oder eingerichtet ist zum Anzeigen und/oder Versenden von Temperaturdaten und darauf basierenden Informationen bevorzugt in Bezug auf das bestimmte Nutztier.

Dadurch umfasst die Vorrichtung alle Elemente, die für eine dauerhafte Überwachung einer größeren Anzahl von Tieren erforderlich ist und diese ist gleichzeitig einfach aufzubauen und/oder bereitzustellen sowiebenutzerfreundlich im Einsatz.

Neben einer festen/fixierten Anbringung an oder benachbart zu der Tierversorgungseinrichtung kommt auch in Frage, einen Temperatursensor vorzusehen, der erst bei Anwesenheit eines Nutztieres in eine Position zum Messen gebracht wird.

Dies kann automatisch geschehen, also z. B. wenn die Anwesenheit des Nutztieres eine Futterausgabe bewirkt wird gleichzeitig beispielsweise ein Verschwenken eines an einem Schwenkarm angeordneten Temperatursensors bewirkt. Oder auch manuell, dass beispielsweise ein Benutzer der ohnehin die Tierversorgungseinrichtung und/oder Tierpflegeeinrichtung bedient und/oder befüllt durch Betätigung oder manuell einen Temperatursensor in die Position zum Messen bringt. Deshalb kann der Temperatursensor erfindungsgemäß auch einer sein, der von einem Benutzer mitgeführt und per Hand bewegt wird.

Insbesondere kann mittels eines gesteuerten Motors an der Halterung für den Temperatursensor, insbesondere das Pyrometer, dieser auf eine bestimmte Stelle des Körpers des Tieres gerichtet werden, deren Lage insbesondere zuvor bestimmt werden kann durch Auswerten eines kurz vor der Temperatur-Messung angefertigten Bildes des Tieres entweder mit einer Wärme-empfindlichen Kamera oder einer normalen Kamera

Die Tierversorgungseinrichtung bzw. Tierpflegeeinrichtung ist dabei abhängig von Tierart und konkreten Haltungsbedingungen wählbar und kann prinzipiell jede Art von Einrichtung sein, die regelmäßig von den Tieren aufgesucht wird. Denn dann ist sichergestellt, dass regelmäßig bei allen Tieren der Herde eine Temperaturmessung stattfinden kann.

In Frage kommen insbesondere Futterstellen/-einrichtungen, Tränken, Melkstationen, Durchgangsbereiche wie beispielsweise von einem abgezäunten/abgetrennten Bereich in einen anderen oder auch von einem Raum zu einem anderen oder der Übergang Außenfläche ins Gebäude oder auch Laderampen für Laster (die von den Tieren wenigstens 2 mal passiert werden, so dass eine Überwachung der Gesundheitsänderung während des Transports leicht möglich ist).

Als Temperatursensoren kommen alle gebräuchlichen in Frage, die eine Oberflächen-Temperatur berührungslos bestimmen können.

Die Auswerteeinheit kann eine entsprechend programmtechnisch eingerichtete Datenverarbeitungseinheit sein. Es kann hier eine eigene zusätzliche vorgesehen sein, aber auch eine bereits bestehende programmtechnisch als erfindungsgemäße Auswerteeinheit eingerichtet werden.

Dadurch, dass die Auswerteeinheit Temperaturdaten bezogen auf ein bestimmtes Nutztier speichert, sind durch die Auswerteeinheit die Temperaturdaten von jedem einzelnen Nutztier der letzten und bevorzugt von allen Messungen und insbesondere bevorzugt in Verbindung mit anderen Daten, wie beispielsweise Zeitpunkt der Messung, verzehrte Futtermenge, abgegebene Milch, etc., verfügbar. Dadurch lassen sich leicht und insbesondere bevorzugt auch automatisiert, bevorzugt unmittelbar durch eine hierfür eingerichtete Auswerteeinheit, Nutztiere, insbesondere auch bei größeren Herden, auffällige Tiere ermitteln, häufig auch bevor sonstige Krankheitssymptome offensichtlich werden.

Die Sende- und/oder Anzeigeeinheit dient dem Zweck, dass Benutzer allgemein oder im Falle von Auffälligkeiten über die Temperaturwerte der Messungen in Kenntnis gesetzt werden, damit diese ggf. erforderliche Schritte einleiten können.

Zu diesem Zweck kann die Sende- und/oder Anzeigeeinheit eine tatsächliche physische Anzeigeeinheit sein, die auch benachbart zu der Auswerteeinheit oder dem Temperatursensor angeordnet sein kann und die eben optisch anzeigt beispielsweise durch Anzeigen der Zahlenwerte oder über Symbolik, welche Temperatur gemessen wurde und/oder ob diese problematisch ist.

Sie kann aber auch primär als Sendeeinheit ausgelegt sein, die beispielsweise entsprechende elektronische Nachrichten generiert, die dann auf entsprechenden Empfangs-Geräten von Nutzern zu einer Anzeige führen, wie beispielsweise Smartphones, Computer, Tablets und/oder ähnliches, welche durch Bluetooth, WLAN, SMS, Mails und/oder ähnliches kontaktiert werden.

Die Sende- und/oder Anzeigeeinheit kann aber auch eine Kombination aus Senden und Anzeigen verwirklichen.

Bevorzugt ist der Temperatursensor ein Pyrometer, insbesondere ein Infrarot-Pyrometer, und/oder eine Wärmebildkamera und/oder ein Infrarotsensor. Diese und ähnliche Sensoren sind hinsichtlich der Anwendung bei Nutztieren unter üblichen Haltungsbedingungen für berührungslose Temperaturmessungen am zuverlässigsten und benötigen gleichzeitig wenig Messzeit, sodass sie beispielsweise auch in einem Durchgangsbereich (z.B. auch Treibgang) zuverlässige Temperaturdaten liefern, in denen sich ein Tier auch nur kurz aufhält, wenn auch regelmäßig.

Ferner können auch Messungen ermöglicht werden, wenn mehrere Tiere anwesend sind. Beispielsweise mit einer Wärmebildkamera, die gleichzeitig mehrere Tiere erfasst, und/oder mit einem automatisch oder vom Benutzer bewegten Pyrometer, welches schnell nacheinander auf die einzelnen Tiere gerichtet wird.

Pyrometer sind als Temperatursensor einer erfindungsgemäßen Vorrichtung besonders geeignet. Diese bestrahlen die zu messende Fläche mit geeigneter Strahlung und messen die in Reaktion von der zu messenden Fläche ausgehende Strahlung, insbesondere deren Wärmestrahlung. Bevorzugt wird eine InfrarotStrahlung verwendet. Es kommt aber auch in Frage Strahlung verschiedener Wellenlänge auszusenden, um anhand der unterschiedlichen Reaktion der zu messende Oberfläche eine genauere Temperaturbestimmung zu erreichen.

Grundsätzlich lassen sich mit Pyrometern mit wenig Aufwand genau die Oberflächen-Temperaturen der Tiere bestimmen.

Bevorzugt umfasst der Temperatursensor ein oder besser mindestens zwei Sensorelemente, die bei Anordnung des Temperatursensors an oder benachbart zur Tierversorgungseinrichtung die Oberflächen-Temperatur an unterschiedlichen Stellen des Körpers des Nutztieres und/oder aus unterschiedlichen Richtungen bezogen auf den Körper des Nutztieres messen. Allgemein ist es bevorzugt mit mindestens einem Sensorelement einige Stellen des Körpers des Nutztieres zu erfassen (z.B. beim stehenden oder gehenden Tier, die für mindestens ein anderes Sensorelement durch den Körper des Nutztieres verdeckt sind.

Bei vielen Nutztieren und entsprechenden Haltungsbedingungen sind gewisse Verschmutzungen der Körperoberfläche nichts Ungewöhnliches. Würde die Temperatur nur an einer verschmutzten Stelle des Tierkörpers gemessen werden, könnte der Schmutz leicht eine Verfälschung des Messergebnisses bewirken.

Dadurch, dass die mindestens zwei Sensorelement jeweils Stellen erfassen, die für mindestens ein anderes Sensorelement verdeckt sind, ist sichergestellt, dass die Sensorelemente an erheblich verschiedenen Stellen messen, die im Allgemeinen auch unterschiedlich verschmutzt sind. Dadurch kann eine einzelne großflächige Verschmutzung das Ergebnis nicht mehr verfälschen bzw. zumindest wird die erhebliche Abweichung des einen Sensorelements registriert. Bei Messung von nur zwei benachbarten Punkten besteht hingegen das Risiko bei einer großflächigen Verschmutzung, dass diese das gleiche fehlerhafte Ergebnis liefern und es dann auch noch mangels Abweichung von einem Normbereich nicht auffällt, obwohl die Temperatur des Tieres in Wirklichkeit außerhalb des Normbereiches liegt.

Ebenfalls kann durch das Vorsehen von mindestens zwei Sensorelementen und einem Abgleich von deren Messergebnissen auch leichter eine Fehlfunktion eines Sensorelementes selbst erkannt werden.

Entsprechend ist hierfür bevorzugt die Auswerteeinheit eingerichtet, problematische Diskrepanzen zwischen den Messungen der mindestens zwei Sensorelemente zu ermitteln und entsprechende Hinweis- und/oder Warnsignale zu erzeugen/zu senden, beispielsweise mittels der Sende- und Anzeigeeinheit.

Bevorzugt sind bei der Nutzung mehrerer Sensorelemente ferner die Sensorelemente mit erheblich verschiedenen Blickwinkeln einzurichten. Beispielsweise zwei Pyrometer und/oder Wärmebildkameras, die von zwei benachbarten Wänden oder Wandungen auf den Bereich dazwischen gerichtet sind, beispielsweise bei einer Futterstelle oder einem Durchgang.

Bevorzugt umfasst die Vorrichtung ferner ein Tieridentifikationsmittel, wobei bevorzugt das Tieridentifikationsmittel einen Scanner, einen Barcode-Scanner, einen RFID-Scanner, einen Ohrmarken-Scanner und/oder eine Kamera umfasst, wobei das Tieridentifikationsmittel bevorzugt eine Identifikationseinheit, bevorzugt eingerichtet zur Gesichtserkennung, umfasst und/oder wobei die Auswerteeinheit gleichzeitig als eine solche Identifikationseinheit des Tieridentifikationsmittels eingerichtet ist und/oder in einem solchen Identifikationsprozess genutzt wird.

Alternativ oder zusätzlich kann die erfindungsgemäße Vorrichtung auch Mittel zur Eingabe und/oder Auswahl einer Tieridentität durch einen Nutzer umfassen. Dies kommt vor allem in Frage, wenn die Tierversorgungseinrichtung und/oder Tierpflegeeinrichtung ohnehin überwacht und/oder manuell betätigt werden muss und der damit zwingend anwesende Nutzer/Bediener das Tier identifizieren kann.

Alternativ oder zusätzlich können auch Tieridentifikationsmittel der Tierversorgungseinrichtung und/oder Pflegeeinrichtung die Tieridentifikationsmittel der erfindungsgemäßen Vorrichtung oder ein Teil davon sein und/oder als solches dienen.

In der Nutztierhaltung werden üblicherweise Nutztiere mit entsprechenden Identifikationselementen wie Ohrmarken versehen, so dass die Zuordnung der Temperaturmessung zu einem bestimmten Nutztier am einfachsten mittels Identifikation des ohnehin vorhandenen Identifikationselements geschieht.

Erfindungsgemäß ist es ebenfalls, wenn ohnehin an der Tierversorgungs- und/oder Tierpflegeeinrichtung vorgesehene Identifikationsmittel als erfindungsgemäße Identifikationsmittel dienen und die Auswerteinheit entsprechend hierfür eingerichtet ist. Denn z. B. kommen ohnehin Futterstellen zum Einsatz, die Futter abhängig vom konkreten Nutztier abgeben und entsprechend ohnehin über Identifikationsmittel verfügen.

Grundsätzlich kann aber auch Gesichtserkennungssoftware erfindungsgemäß zum Einsatz zu kommen. Weshalb erfindungsgemäß auch eine Kamera als Tieridentifikationsmittel bzw. als ein Element davon dienen kann. Es ist erfindungsgemäß auch denkbar, dieselbe Kamera als Tieridentifikationsmittel und als Temperatursensor zu nutzen, beispielsweise indem die Identifikation anhand des Infrarotbildes erfolgt oder durch Nutzung einer sowohl infrarotes als auch sichtbares Licht aufzeichnenden Kamera.

Bevorzugt umfasst die Sende- und/oder Anzeigeeinheit mehrere bevorzugt mehrfarbige Leuchtmittel, insbesondere Leuchtdioden, und/oder mindestens einen Lautsprecher zur Erzeugung unterschiedlicher Signaltöne, wobei den Leuchtmitteln bzw. den Signaltönen jeweilige Temperaturbereiche einer gemessenen Oberflächen-Temperatur in eindeutiger Weise zugeordnet sind und/oder wobei jeweiligen Temperaturbereichen in eindeutiger Weise bestimmte Warnleuchtmittel, Warnfarben und/oder Warnsignaltöne zugeordnet sind, bevorzugt so, dass eine außerhalb eines Toleranzbereiches liegende Oberflächen-Temperatur anzeigbar ist.

Dabei kann vorgesehen sein, dass Leuchtmittel bzw. Lautsprecher bei jeder Temperaturmessung grundsätzlich aktiv werden, also beispielsweise auch entsprechende optische und/oder akustische Signale erzeugen, wenn eine Messung unauffällig ist.

Dies kann beispielsweise bei Leuchtmitteln in Form einer Ampel mit grünen, gelben und roten Leuchtmitteln erfolgen. Eine Messung in einem Normbereich führt zu einem grünen Licht, wodurch ein Nutzer sieht, insbesondere auch auf weite Distanz durch einen Stall hindurch, dass bei dem gerade vermessenen Nutztier die Temperatur in einem Normalbereich ist (bzw. in Abhängigkeit von vorherigen Messwerten unauffällig ist, z. B. bei einem Tier mit gerade abklingender Krankheit kann ein reines Absinken der Temperatur bereits ausreichend sein, dass keine weiteren Maßnahmen erforderlich sind).

Ein gelbes Licht wird beispielsweise erzeugt, wenn die Messwerte es anzeigen, dass ein Nutztier noch einmal optisch durch den Nutzer/Bediener der Anlage geprüft werden soll. Und ein rotes Licht wird beispielsweise erzeugt, wenn die Temperatur des Nutztieres so ist, dass unmittelbar Maßnahmen erfolgen müssen, wie beispielsweise eine Separierung des Nutztieres, weitergehende Untersuchungen oder andere Maßnahmen und/oder die Hinzuziehung eines Veterinärmediziners.

Da eine solche quasi als Ampel ausgebildete Sende- und/oder Anzeigeeinheit weithin im Stall oder der Anlage sichtbar ausgebildet sein kann, ist sichergestellt, dass bei Nutztieren mit entsprechenden Problemen zeitnah geeignete Maßnahmen durch anwesende Nutzer/Bediener erfolgen, aber gleichzeitig jeweils eine sichtbare optische Bestätigung erfolgt, wenn ein Nutztier nicht auffällig ist.

Ferner ist natürlich auch denkbar, Blau, Grün und Rot oder andere Farben oder Muster zu nutzen, um eine zu niedrige Temperatur, eine Normaltemperatur und eine zu hohe Temperatur anzuzeigen.

Alternativ kann aber auch vorgesehen sein, dass nur bei entsprechender Auffälligkeit ein Signal erzeugt wird. Beispielsweise, dass ein akustisches Warnsignal ausgegeben wird, wenn die Temperatur eines Nutztieres auf eine für die Herde gefährliche ansteckende Krankheit hindeutet und deshalb eine sofortige Separierung zwingend ist. Ist hingegen eine Messung unauffällig oder zumindest nicht besonders problematisch, erfolgt kein akustisches Warnsignal (aber beispielsweise eine elektronische Nachricht, dass bei Gelegenheit ein leicht auffälliges Nutztier zu überprüfen ist).

Es lässt sich auch beides kombinieren, also beispielsweise die zuvor geschilderte Ampelvariante mit einem akustischen Warnsignal bei besonders problematischen Temperaturen.

Sowohl die optische als auch die akustische Anzeige kann auch komplexer sein, beispielsweise, dass Farbe/Helligkeit/Tonhöhe/Lautstärke/Anzahl Lichter oder Töne oder ein anderer entsprechender Parameter mit dem Messwert korreliert ist. Beispielsweise eine Lichtskala, von der umso mehr Lichter beginnend von einem Ende aus leuchten, je höher die Temperatur ist, und mit sich ändernder Lichtfarbe im Verlauf der Skala, beispielsweise von Grün nach Rot. Damit wird quasi eine skalierte Ampel realisiert, die in weithin sichtbaren Signalfarben Grün/Gelb/Rot die allgemeine Einordnung der Messung anzeigt, an der aber bei näherer Betrachtung unmittelbar auch der gemessene Temperaturwert abgelesen werden kann.

Ebenfalls kann bei der vorher geschilderten Nutzung von mehreren Sensorelementen als Temperatursensor vorgesehen sein, mehrere separate Leuchtmittel/Lautsprecher vorzusehen, die jeweils einzelnen oder bestimmten Gruppen von Sensorelementen zugeordnet sind. Damit kann dann beispielsweise weithin sichtbar jeweils das Messergebnis von beiden Seiten des Tieres her angezeigt werden.

Bevorzugt sind der Temperatursensor, die Auswerteeinheit, das Tieridentifikationsmittel und/oder die Sende- und/oder Anzeigeeinheit mit Kabeln und/oder Steckern und/oder elektronisch und/oder per Fernkommunikation verbunden und/oder integral ausgebildet und/oder als eine Einheit ausgebildet, bevorzugt verbunden mit oder als Teil eines Handhabungsmittels, welches es einem Nutzer erlaubt, den Temperatursensor an oder benachbart zu der Tierversorgungseinrichtung und/oder Tierpflegeeinrichtung anzuordnen und/oder zu positionieren.

Es können alle technisch bekannten Varianten zur Verbindung/Ausbildung der unterschiedlichen Komponenten genutzt werden. Die konkrete Wahl richtet sich meist nach der konkreten Ausgestaltung der Nutztierhaltungsanlage bzw. des Stalls und den vorhandenen Systemen. Beispielsweise verfügen viele Ställe bereits über elektronische Überwachung für die bereits in der Praxis genutzten Temperaturmessvorrichtungen, womit eine erfindungsgemäße Vorrichtung dann entsprechend in das bestehende System integriert werden kann.

Eine solche Integration ist gerade dann einfach möglich, wenn einige oder alle entsprechenden noch nicht im bestehenden System vorhandenen Elemente des erfindungsgemäßen Systems als Teil eines Handhabungsmittels ausgebildet werden. Durch elektronische Ankoppelung an das bestehende System ist dann zur Realisierung einer erfindungsgemäßen Vorrichtung nicht mehr als das Handhabungsmittel mit entsprechenden integrierten erfindungsgemäßen Komponenten (beispielsweise der Temperatursensor), die Einrichtung des bestehenden Systems entsprechender anderer erfindungsgemäßen Komponenten (beispielsweise programmtechnische Einrichtung einer bestehenden Datenverarbeitungseinheit als erfindungsgemäße Auswerteeinheit und Einrichtung eines bestehenden Informationssystems als erfindungsgemäße Sende- und/oder Anzeigeeinheit hinsichtlich der gemessenen Temperatur) und elektronische Ankoppelung des Handhabungsmittels an das bestehende System (also beispielsweise Vorsehen von entsprechenden WLAN- oder Bluetooth-Mitteln am Handhabungsgerät und Verbindung mit dem bestehenden System).

In einer bevorzugten Variante der integralen Ausbildung ist der Temperatursensor als Aufsteckeinheit und/oder Teil einer Aufsteckeinheit ausgebildet, insbesondere zum Aufstecken und/oder Verbinden mit einem Smartphone, wobei das Smartphone besonders bevorzugt eingerichtet ist als Auswerteeinheit und/oder Sende- und/oder Anzeigeeinheit, wobei bevorzugt die Stromversorgung des Temperatursensors über die Verbindung mit dem Smartphone oder mittels Batterie/Akku erfolgt.

Da Smartphones ohnehin weitverbreitet sind, kann damit eine erfindungsgemäße Vorrichtung mit minimalen Hardwareanpassungen genutzt werden. Beispielsweise indem ein Nutzer bei einer ohnehin regelmäßig stattfindenden optischen Inspizierung/Durchzählung der Nutztiere sein zuvor per App eingerichtetes Smartphone mit einer entsprechenden Aufsteckeinheit versieht und auf jeweils jedes Nutztier nacheinander ausrichtet, z.B. indem er es in einer fest installierten Halterung mit vorzugsweise immer gleicher Ausrichtung fixiert.

Ein eigenständiges mobiles Gerät kann auch genutzt werden zur Ergänzung der stationären Vorrichtung oder auch alleine Messungen durchzuführen. Dadurch ist eine höhere Flexibilität möglich.

Auch können die Daten über das Smartphone leicht weitergeleitet werden.

Darüber hinaus können auch andere Eigenschaften des Smartphones genutzt werden. So kann die Verbindung auch genutzt werden, um Daten an die Auswerte-Einheit zu senden und gegebenenfalls von der Auswerte-Einheit Ergebnisse oder weiterführende Informationen zu erhalten.

Darüber hinaus können auch verschiedene Applikationen des Smartphones genutzt werden zum Beispiel zur Tiererkennung (Foto vom Tier, von der Ohrmarke, Lesen des Barcodes, QR-Code und andere, insbesondere optische, Markierungen.

Bei Vorhandensein einer Zeitleiste können die Daten von Foto und Messwert auch eindeutig zeitlich zugeordnet werden.

Ferner kann bevorzugt die Aufsteckeinheit auch weitere und/oder alle der zuvor oder nachfolgend genannten Komponenten der erfindungsgemäßen Vorrichtung umfassen. Beispielsweise eine Aufsteckeinheit mit einer skalierten aus LEDs gebildeten Ampel als Teil der oder als die Sende- und Anzeigeeinheit.

Bevorzugt ist der Temperatursensor eingerichtet, auf Anstieg einer gemessenen Temperatur, Aktivierung der Tierversorgungseinrichtung und/oder Tierpflegeeinrichtung oder auf Ansprechen eines mit dem Temperatursensor verbundenen oder darin integrierten Bewegungssensors und/oder Mikrophons eine Messung der Oberflächen-Temperatur und/oder eine gemessene Oberflächen-Temperatur an die Auswerteeinheit zu senden.

Dadurch kann durch geeignete Wahl des Auslösungsmechanismus sichergestellt werden, dass der Temperatursensor nur dann das Temperatursignal misst bzw. weiterleitet, wenn tatsächlich ein Nutztier anwesend ist und bevorzugt in der richtigen Position. Denn idealerweise sollten die Messungen jeweils an ähnlichen oder gleichen Stellen des Tierkörpers erfolgen.

Alternativ oder zusätzlich kann aber auch ein Teil dieser Funktionalität auf der Auswerte-Einheit realisiert sein. Also, dass beispielsweise der Temperatursensor zwar kontinuierlich misst und die Daten an die Auswerteinheit weiterleitet, aber dass diese dies erst als Messung der Temperatur eines Nutztieres speichert/verarbeitet, wenn eine entsprechende Auslösebedingung/ein entsprechender Auslösemechanismus ebenfalls anspricht.

Bevorzugt ist die Auswerteeinheit dazu eingerichtet, basierend auf Oberflächen-Temperaturen eines bestimmten Nutztieres Folgemaßnahmen auszulösen, wie beispielsweise Aktivierung oder angepasste Aktivierung der Tierversorgungseinrichtung und/oder Tierpflegeeinrichtung und/oder Warnsignalen, insbesondere optischen oder akustischen oder elektronischen, und/oder Änderung/Markierung eines Datenbankeintrags des bestimmten Nutztieres und/oder Benachrichtigungen, insbesondere elektronischen, und/oder Verabreichung von besonderen Tierpflegemitteln wie Medikamenten und/oder Umleitung des Nutztieres in einen speziellen Pflegebereich.

Dadurch lassen sich bei einigen Nutztierarten übliche Standardmaßnahmen bei Auffälligkeiten automatisiert realisieren. Hierbei kann die Auswerteeinheit insbesondere auch eingerichtet sein, die Folgemaßnahmen in Abhängigkeit von anderen Daten neben aktuellen und früheren Temperaturen auszulösen, beispielsweise indem die Auswerteeinheit kombiniert den Temperaturverlauf, Futterverzehr und Milchabgabe eines Nutztieres einbezieht.

Bevorzugt umfasst die erfindungsgemäße Vorrichtung ferner eine Datenverarbeitungseinheit zur Speicherung und Pflege eines Nutztierdatenbestandes, bevorzugt als Zentralstation, wobei die Sende- und/oder Anzeigeeinheit ferner eingerichtet ist, Temperaturdaten mit Referenz auf das zugehörige bestimmte Nutztier an die Datenverarbeitungseinheit und/oder Zentralstation zu senden, wobei die Datenverarbeitungseinheit und/oder Zentralstation dazu eingerichtet ist, Befehlssignale an die Tierversorgungseinrichtung und/oder Tierpflegeeinrichtung, die Auswerteeinheit und/oder die Sende- und Anzeigeeinheit zu senden, um anhand des Nutztierdatenbestandes als erforderlich ermittelte Vorgänge auszulösen.

Dies kann in ähnlicher Form wie die zuvor geschilderte Einrichtung der Auswerteeinheit zur Auslösung von Folgemaßnahmen realisiert sein. Insbesondere können für alle genannten Aufgaben/Vorgänge je nach konkreter Realisierung ganz oder teilweise oder auch gleichzeitig Zentralstation und/oder Auswerteinheit entsprechend eingerichtet sein.

Beispielsweise kommt es bei Nutzung von Aufsteckeinrichtungen auf Smartphones und mehreren Mitarbeitern in einer Anlage in Frage, dass die Aufsteckeinheit bzw. das Smartphone selbst als Auswerteeinheit hauptsächlich nur dahingehend eingerichtet sind, die gemessenen Temperaturdaten an eine Datenverarbeitungseinheit der Anlage als Zentralstation weiterzuleiten, da eine Auswertung auf jeweils der Aufsteckeinheit bzw. dem Smartphone der einzelnen Mitarbeitern hinsichtlich einer Einheitlichkeit der Daten schwierig sein kann.

Es kann aber auch sinnvoll sein, um eine zu große - vor allem für den entsprechenden Nutzer - Datenmenge an der Zentralstation zu vermeiden, einen erheblichen Teil der einfachen Funktionalitäten, wie beispielsweise Auslösung von Warnsignalen hinsichtlich einer akuten Gefährdung und/oder der Futtermengenabgabe, durch entsprechende Einrichtung mit der Auswerteinheit zu realisieren, während die Zentralstation hauptsächlich der längerfristigen Datenpflege dient.

Bevorzugt ist die Auswerteeinheit dazu eingerichtet, in Abwesenheit eines Nutztieres Messungen von Neutraltemperaturen insbesondere einer Testfläche auszuführen bzw. auszulösen, und bei ungewöhnlichen Neutraltemperaturen, insbesondere in Anbetracht einer Raumtemperatur, welche beispielsweise an die Auswerteeinheit regelmäßig von einem Raumtemperatursensor übermittelt wird, ein Störungssignal auszulösen oder zu übertragen, insbesondere an die Sende- und Anzeigeeinheit.

Unter Neutraltemperatur ist hierbei eine Temperaturmessung in Abwesenheit eines Nutztieres oder anderer beweglicher Objekte zu verstehen (= Kalibrierung). Dabei richtet sich die gemessene Temperatur im Allgemeinen nach der Raumtemperatur bzw. der Raumtemperatur der vergangenen Stunden oder Minuten sowie nach der Oberfläche, auf die der Temperatursensor ausgerichtet ist.

Da der Temperatursensor eine feste Messposition in Bezug auf die Tierpflege- und/oder Tierversorgungseinrichtung aufweisen sollte, ist in dieser Position im Allgemeinen der Temperatursensor häufig oder immer auf die gleiche Oberfläche ausgerichtet.

Damit ist durch den Verlauf der Raumtemperatur und der Art der Oberfläche und des entsprechenden Körpers bekannt, welche Temperatur der Temperatursensor messen sollte, womit durch Messungen von solchen Neutraltemperaturen der Temperatursensor regelmäßig überprüft und/oder geeicht oder kalibriert werden kann. Bei entsprechender Abweichung der Neutraltemperatur von einem erwarteten Wert und/oder bei zu starker Schwankung der Neutraltemperatur beispielsweise von einem Tag auf den nächsten, kann ein entsprechender Hinweis erfolgen, dass der Temperatursensor zu überprüfen ist.

Entsprechende Fehler können bereits auftreten, wenn der Temperatursensor verschmutzt wird, was aufgrund der räumlichen Nähe zu den Nutztieren denkbar ist.

Bevorzugt wird sogar eine hinsichtlich Material und Oberfläche speziell ausgewählte Testfläche vorgesehen, auf die der Temperatursensor in der Messposition ausgerichtet ist, so dass die Oberflächen-Temperatur dieser Testfläche vom Temperatursensor gemessen werden kann, wenn kein Nutztier anwesend ist. Es kommt auch in Frage, die Testfläche beweglich auszugestalten und entsprechend für Neutraltemperaturmessungen in den Messbereich des Temperatursensors hineinzubewegen.

Mittels entsprechender Anordnung bzw. beweglicher Anordnung ist die Testfläche bevorzugt so vorgesehen, dass eine Verschmutzung durch die Nutztiere oder auf anderweitige Art unwahrscheinlich oder ausgeschlossen ist. Dadurch ist dann sichergestellt, dass eine fehlerhafte Neutraltemperatur nahezu nur dann auftritt, wenn der Temperatursensor ein Problem aufweist.

Auch sind Testfläche und/oder Temperatursensor bevorzugt so angeordnet, dass sie bei üblicher Reinigung des entsprechenden Bereichs ebenfalls leicht mitgereinigt werden können bzw. automatisch mitgereinigt werden oder eine eigene Reinigungsvorrichtung aufweisen.

Bevorzugt ist der Temperatursensor dazu eingerichtet, eine Entfernung zwischen Temperatursensor und der mindestens einen Stelle des Körpers des Tieres bzw. Nutztieres zu erfassen bzw. zu ermitteln, wobei bevorzugt die Auswerteeinheit und/oder die Datenverarbeitungseinheit ferner dafür ausgelegt ist, die Entfernung zu speichern, zu katalogisieren und/oder eine korrigierte Temperatur unter Verwendung von wenigstens der gemessenen Oberflächen-Temperatur und der Entfernung zu bestimmen. Alternativ oder zusätzlich können aber auch weitere Komponenten separat für die Entfernungsbestimmung vorgesehen sein, beispielsweise indem die erfindungsgemäße Vorrichtung auch einen Entfernungssensor umfasst.

Die Abstandsmessung kann dabei beispielsweise über Wegzeitmessungen erfolgen, insbesondere auch direkt anhand von vom Temperatursensor ausgesandter Strahlung. Aber auch eine optische Bestimmung beispielsweise über ein von einer Kamera erfasstes Bild ist möglich. Ferner können auch Drucksensoren oder Bewegungssensoren zur Bestimmung des Abstandes bzw. der Entfernung genutzt werden, wobei erfindungsgemäß die Bestimmung nicht zwingend mit hoher Genauigkeit erfolgen muss.

Ebenfalls kann alternativ oder zusätzlich zu Entfernungsmessvorrichtungen vorgesehen sein, dass ein Nutzer aufgefordert wird, die Entfernung abzuschätzen und einzugeben.

Die ermittelte Entfernung kann dabei in verschiedener Weise genutzt werden. Beispielsweise zur direkten Korrektur der gemessenen Oberflächen-Temperatur. Beispielsweise ebenfalls als Daten hinsichtlich des Nutztieres, denn Bewegung und Körperhaltung des Nutztieres sind einerseits mit dem Gesundheitszustand korreliert und andererseits mit der Entfernung zum Temperatursensor. Beispielsweise um Unterschiede und Fehler zu erfassen, wenn beispielsweise ein bestimmtes Nutztier meist nah am Temperatursensor steht, aber dann eine Temperaturmessung bei größerer Entfernung erfolgt. Es können so auch systematische Abweichungen oder Fehler besser erkannt werden, beispielsweise, wenn bei größerer Entfernung im Mittel eine niedrigere oder höhere Temperatur gemessen wird als bei kleinerer Entfernung.

Ein erfindungsgemäßer Bausatz und/oder Baukasten zur Ausbildung einer Vorrichtung zur berührungslosen Ermittlung der Körpertemperatur eines Tieres bzw. Nutztieres umfasst:
- einen Temperatursensor zur Messung der Oberflächen-Temperatur an mindestens einer Stelle des Körpers des Nutztieres;
- eine Verbindungseinrichtung zum Verbinden oder benachbart Anordnen des Temperatursensors mit bzw. zu einer Tierversorgungseinrichtung und/oder Tierpflegeeinrichtung;
- eine Auswerteeinheit, die mit dem Temperatursensor für einen Datenaustausch verbindbar ist, insbesondere durch Fernkommunikation, und die zum Erfassen der von dem Temperatursensor gemessenen Oberflächen-Temperatur ausgelegt ist, und/oder einen Datenträger, der mit Programmdaten eingerichtet ist zur Einrichtung von einer im Umfeld der Tierversorgungseinrichtung und/oder Tierpflegeeinrichtung vorhandenen Datenverarbeitungseinheit als Teil der oder insgesamt als Auswerteeinheit; und
- eine Sende-, Empfänger- und/oder Anzeigeeinheit, die mit der Auswerteeinheit für einen Datenaustausch verbindbar ist oder integral mit dieser ausgebildet ist und/oder den bzw. einen Datenträger, der mit Programmdaten eingerichtet ist zur Einrichtung von einem im Umfeld der Tierversorgungseinrichtung und/oder Tierpflegeeinrichtung vorhandenen Datenverarbeitungseinheit als Teil der oder insgesamt als Sende- und/oder Anzeigeeinheit,
   wobei der Bausatz/Baukasten ausgelegt ist, durch Anordnung und/oder Installation an oder bei einer Tierversorgungseinrichtung und/oder Tierpflegeeinrichtung eine erfindungsgemäße Vorrichtung auszubilden.

Mittels eines solchen erfindungsgemäßen Bausatzes kann problemlos in bestehenden Anlagen eine erfindungsgemäße Vorrichtung ausgebildet bzw. eingerichtet werden.

Bevorzugt kann der Bausatz zur Realisierung einer oder mehrerer der zuvor geschilderten bevorzugten Weiterbildungen der erfindungsgemäßen Vorrichtung ausgebildet sein bzw. zur Realisierung einiger oder aller Merkmale von mehreren der genannten Weiterbildungen der erfindungsgemäßen Vorrichtung ausgebildet sein. Der Bausatz kann dann entsprechende Komponenten umfassen, wie beispielsweise mehrere Leuchtmittel, die beispielsweise als skalierte Ampel ausgebildet sind und/oder eine Datenverarbeitungseinheit als Zentralstation und/oder einen Datenträger mit Programmdaten zum Einrichten einer Datenverarbeitungseinheit als Zentralstation.

Ein erfindungsgemäßes Verfahren, welches bevorzugt bei Nutzung einer erfindungsgemäßen Vorrichtung in einer der geschilderten Varianten oder einer durch einen erfindungsgemäßen Bausatz ausgebildeten Vorrichtung ausgeführt wird bzw. auf bzw. mit einer solchen Vorrichtung ausgeführt wird, umfasst die Schritte:
berührungsloses Messen der Oberflächen-Temperatur eines Tieres bzw. Nutztieres bevorzugt mittels einer erfindungsgemäßen Vorrichtung;
Identifizieren des Nutztieres;
Abgleich, Vergleich und/oder Wertung der gemessenen Oberflächen-Temperatur mit bzw. anhand von früher gemessenen Oberflächen-Temperaturen desselben Nutztieres und insbesondere an derselben Messstelle oder denselben Messstellen (Pl);
Auslösung einer Folgefunktion abhängig von dem Ergebnis von Abgleich, Vergleich und/oder Wertung; und
Speicherung der gemessenen Oberflächen-Temperatur unter Referenz auf das zugehörige Nutztier und bevorzugt in Verbindung mit anderen Daten wie Uhrzeit, Datum, Fütterungsmenge, verstrichene Zeit seit dem letzten Aufsuchen der Einrichtung durch das Tier (und Vergleich mit der normalerweise zwischen 2 Besuchen verstrichenen durchschnittlichen Zeit), Verweildauer des Nutztieres in der Tierversorgungseinrichtung und/oder Tierpflegeeinrichtung, Menge der gemolkenen Milch und/oder anderer für die Tierhaltung relevanter Daten;
wobei alle Schritte mindestens einmal pro Woche, bevorzugt mindestens einmal pro 24 Stunden ausgeführt werden.

Das erfindungsgemäße Verfahren kann bevorzugt auch jeweils die Nutzung von Elementen bzw. Schritte entsprechend der zuvor hinsichtlich der erfindungsgemäßen Vorrichtung bzw. hinsichtlich des erfindungsgemäßen Bausatzes geschilderten bevorzugten Weiterbildungen umfassen. Beispielsweise, dass der Schritt des Messens mehrere Unterschritte in Form von Messen aus verschiedenen Richtungen bzw. von verschiedenen Punkten der Oberfläche des Nutztieres umfasst. Oder auch beispielsweise einen Schritt mit einer Eichungsmessung der Oberflächen-Temperatur einer Testfläche.

Vorzugsweise werden eine größere Anzahl von Messungen, insbesondere mindestens 5 Messungen, besser mindestens 10 Messungen, hintereinander in relativ kurzer Zeit, insbesondere in weniger als 30 s, besser weniger 20 s, besser weniger 10 s besser weniger 5 s an einem Tier durchgeführt und insbesondere ein Höchstwert (max), Mittelwert oder sonstiger standardisierter Wert, insbesondere ein Mittelwert, dieser Temperaturen als aktueller Messwert genommen.

Ebenso können von möglichst vielen, vorzugsweise allen Tieren, einer Gruppe oder einer Herde innerhalb eines kurzen Zeitraumes, zum Beispiel von 2 Tagen oder nur einem Tag, die Temperaturen gemessen werden und daraus als Mittelwert eine Herden-Temperatur ermittelt werden, die zum Vergleich mit der im gleichen Zeitraum gemessenen Temperatur eines konkreten Tieres dient und bei zu starker Abweichung von der Herden-Temperatur automatisch reagiert wird, insbesondere ein Alarmsignal abgegeben wird.

Bevorzugt werden die Schritte in der zuvor genannten Reihenfolge ausgeführt, wobei eine beliebige andere Reihenfolge und auch jeweils gleichzeitige Ausführung ebenfalls erfindungsgemäß ist, insbesondere eine andere Reihenfolge von Messen der Oberflächen-Temperatur eines Tieres bzw. Nutztieres und Identifizieren des Nutztieres und insbesondere auch gleichzeitiges Messen und Identifizieren.

Vorrichtung, Bausatz und Verfahren der Erfindung betreffen Einzelmessungen bevorzugt in Verbindung mit der Zuordnung zu dem jeweiligen Tier bzw. Nutztier.

Die Zuordnung kann bei der erfindungsgemäßen Vorrichtung, bei dem erfindungsgemäßen Bausatz und/oder bei dem erfindungsgemäßen Verfahren beispielsweise durch manuelle Eingabe des Namens oder einer Nummer durch den Tierhalter oder Betreuer oder durch Lesen von Barcodes (z.B. auf der Ohrmarke) oder Scannen eines RFID Signales des Tieres (z. B. von Transponder) oder mittels Bilderkennung (Biometrie) erfolgen.

Zwar ist die Erfindung vor allem in Bezug auf die Nutztierhaltung geschildert. Aber erfindungsgemäß können Vorrichtung, Bausatz und/oder Verfahren der Erfindung auch hinsichtlich anderer Tiere, beispielsweise Wildtieren oder auch Haustieren, zum Einsatz kommen.

Beispielsweise werden bei der Überwachung von Wildtierbeständen ohnehin häufig quasi künstliche Futterstellen eingerichtet und per Kamera oder ähnlichem das Auftauchen eines Wildtieres an entsprechender Stelle registriert, um beispielsweise die Anzahl der Wildtiere zu überwachen.

Eine entsprechend eingerichtete künstliche oder auch natürliche Futter- oder Tränkstelle ist ebenfalls eine Tierversorgungseinrichtung und/oder Tierpflegeeinrichtung im Sinne der Erfindung und kann entsprechend für Vorrichtung, Bausatz und/oder Verfahren der Erfindung genutzt werden oder ein Element davon sein.

Im gleichen Sinne ist auch eine Futterstelle eines Haustieres oder Heimtieres eine Tierversorgungseinrichtung und/oder Tierpflegeeinrichtung im Sinne der Erfindung und kann entsprechend für Vorrichtung, Bausatz und/oder Verfahren der Erfindung genutzt werden oder ein Element davon sein.

Zur Temperaturerfassung werden bevorzugt Infrarotmessungen bzw. -strahlung genutzt, denn die Körperkerntemperatur, die Körperinnentemperatur und die Körperoberflächentemperatur ist korreliert und zwar vom Grundsatz her im Sinne einer konstanten Temperaturdifferenz, wobei es Abweichungen geben kann (beispielsweise variierende Felldicke, Verschmutzungen der Körperoberfläche, Umgebungstemperatur etc.). Abweichungen lassen sich jedoch durch geeignete Algorithmen leicht feststellen und eliminieren, wofür die Auswerteeinheit und/oder die Zentralstation entsprechend eingerichtet sein können.

Die Abweichungen der gemessenen Temperatur von der Körperinnentemperatur ist bei einigen bevorzugten Stellen des Körpers besonders gering, beispielsweise beim Messen der Temperatur auf der Oberfläche der Vorderseiten der Ohren und - besonders bevorzugt - beim Messen der Temperatur des Auges.

Um insbesondere an diesen beiden Körperstellen messen zu können, werden diese als Messpunkt und damit Zielpunkt eines an einer konkreten Stelle messenden Temperatur-Sensors wie eines Pyrometers dadurch automatisch ermittelt und können durch das Pyrometers automatisch angezielt werden, indem mittels z.B. Gesichtserkennung, insbesondere von vorne, das Gesicht des Tieres abfotografiert wird und mittels Bildauswertung, insbesondere unterstützt von künstlicher Intelligenz, die Gesichtsform und die Lage des gewünschten Zielpunkt ermittelt wird und der Pyrometer automatisch darauf eingestellt wird.

Darüber hinaus kann aufgrund der Größe des Abbildes vom Gesicht verglichen mit vorher oder gleichzeitig ermittelten Originalabmessungen des Gesichts der Abstand der Kamera, insbesondere Frontkamera und damit auch des Temperatursensors vom Gesicht und damit dem des Zielpunktes ermittelt werden, und die gemessene Temperatur abhängig von der ermittelten Entfernung korrigiert werden.

Auch wenn als Temperatursensor ein flächiger Wärmesensor benutzt wird, der ein Wärmebild einer ganzen Körperpartie wie etwa des Gesichts oder der Seite des Kopfes anfertigt, kann durch Bildauswertung wie oben dargelegt diejenige Stelle des Wärmebildes ermittelt werden, die der gewünschten Messstelle entspricht und die Temperatur dieses Bereichs im Wärmebild als Messwert ausgewählt werden und/oder darauf der punktuell messende Wärmesensor wie ein Pyrometer gerichtet werden und messen.

Vorteil von Vorrichtung, Bausatz bzw. Verfahren der Erfindung ist insbesondere, dass die rektale Temperaturmessung von Hand dank Reduzierung der Umstellungshindernisse und -hemmnisse abgelöst werden kann. Es ist auch kein Nachteil bei der Präzision zu befürchten, da die erfindungsgemäß ermittelte Oberflächen-Temperatur in Korrelation zur rektal bestimmten Temperatur steht.

Die Vorrichtung kann zudem kontinuierlich Daten liefern. Die Temperaturmessung ist auch über längere Zeiträume möglich. Dabei kann eine größere Zahl an Tieren gleichzeitig überwacht werden. Dadurch kann als Mittelwert der bei den einzelnen Tieren gemessenen Temperaturen eine Herden-Temperatur ermittelt werden, insbesondere über einen von der letzten Messung zurückreichenden, festgelegten Zeitraum eine rollierende Herdentemperatur, die dann viele, aber in der Regel nicht alle, Tiere erfasst, wenn der festgelegte Zeitraum relativ kurz ist, beispielsweise ein Tag oder weniger als ein Tag.

Die Temperatur kann ferner direkt visualisiert werden. Die Temperaturdaten können auch automatisch per Fernkommunikation übermittelt werden und insbesondere extern ausgewertet werden.

Vorrichtung, Bausatz bzw. Verfahren der Erfindung ermöglichen es jeweils, eine größere Anzahl von Tieren ohne großen Arbeitsaufwand lückenlos zu überwachen, so dass gezielt (individuell) eingegriffen werden kann, bevor sichtbare Krankheitssymptome auftreten. Das minimiert den Bedarf an Medikamenten - insbesondere Antibiotika - und trägt somit auch dazu bei, Resistenzbildung zu vermeiden.

Überdies werden für den Tierhalter bzw. Nutzer die Behandlungskosten minimiert und nicht zuletzt dienen weniger Medikamente auch dem Verbraucherschutz.

Es sind in der geschilderten und auch auf andere Weise einfach Weiterbildungen der Erfindung möglich. Z. B. kann wie geschildert die gemessene und an eine Zentralstation übermittelte Temperatur im Falle dessen, dass sie einen vorgegebenen Toleranzrahmen über- oder unterschreitet, direkt an den Nutzer, an den Landwirt oder an den Tierarzt übermittelt werden (beispielsweise per E-Mail), so dass hier weitere Untersuchungen oder diagnostische Maßnahmen ansetzen können.

Mittels eines Gerätes mit integriertem Infrarotsensor als Temperatursensor kann punktuell die Temperatur gemessen werden. Bei Tieren kann damit die Oberflächentemperatur ermittelt werden.

Mit einer Infrarotkamera als Temperatursensor kann flächenhaft die Temperatur ermittelt und ein sogenanntes Wärmebild erzeugt werden. Damit können viele Temperaturmesspunkte bestimmt werden.

Mittels eines sogenannten Referenz-Strahles, erzeugt beispielsweise durch den Temperatursensor und/oder eine dazu gehörige oder damit verbundene Komponente, kann die Temperatur rechnerisch korrigiert werden und so die Genauigkeit der Temperaturmessung gesteigert werden.

Wie geschildert können die Sende- und/oder Anzeigeeinheit zum drahtlosen Senden des Temperatursignals ausgebildet sein. Die Sende- und/oder Anzeigeeinheit kann wie geschildert auch zum optischen Anzeigen des Temperatursignals ausgebildet sein.

Die Sende- und/oder Anzeigeeinheit kann wie geschildert eine Anzahl Leuchtdioden aufweisen, denen jeweilige Temperaturbereiche des gemessenen Temperatursignals zugeordnet sind. Insbesondere kann vorgesehen sein, dass die Sende- und/oder Anzeigeeinheit verschieden farbige Leuchtdioden für drei unterschiedliche Temperaturbereiche aufweist, beispielsweise entsprechend der zuvor erläuterten Ampel. Oder auch eine Leuchtdiode für zu hohe, eine für zu niedrige und eine für eine normale Temperatur.

Der Temperatursensor ist bevorzugt so ausgebildet, dass er für die zu erwartenden Temperaturen (bei Säugetieren zwischen 25°C und 45°C) optimal geeignet ist.

Für die Fernkommunikation werden bevorzugt drahtlose Datenübertragungselemente vorgesehen bzw. verwendet, die über RFID an einer Zentralstation bzw. Datenverarbeitungseinheit angemeldet werden können. Die hierfür benötigten RFID-Transponder sind in diesem Falle bevorzugte Bestandteile der erfindungsgemäßen Vorrichtung bzw. des erfindungsgemäßen Bausatzes. Die Zentralstation überwacht gegebenenfalls eine große Anzahl derartiger Sender. Diese sind mit einer geeigneten Energiequelle versehen. Hierfür gegebenenfalls vorgesehene aufladbare Akkumulatoren oder Batterien sind so ausgelegt, dass ein hinreichend langer Betrieb gewährleistet ist.

Teil der Sende- und/oder Anzeigeeinheit kann ebenfalls eine über Funk mit einer Sendestation der Sende- und/oder Anzeigeeinheit in Verbindung stehende Empfangsstation sein, die zum Empfang des von der Sende-und/oder Anzeigeeinheit gesendeten Temperatursignals ausgebildet ist.

Eine bevorzugte Weiterbildung ist hierbei, dass die Empfangsstation in der erläuterten Weise Mittel zum optischen und/oder akustischen Anzeigen des empfangenen Temperatursignals aufweist, also beispielsweise die erläuterten Leuchtmittel und/oder Lautsprecher. Dabei kann insbesondere vorgesehen werden, dass die Mittel der Empfangsstation zum optischen Anzeigen des empfangenen Temperatursignals in der bereits erläuterten Weise eine Anzahl Leuchtdioden aufweist, denen jeweilige Temperaturbereiche des empfangenen Temperatursignals zugeordnet sind. Besonders bevorzugt sind drei verschieden farbige Leuchtdioden für drei unterschiedliche Temperaturbereiche vorgesehen, insbesondere in den Farben Grün, Gelb/Blau und Rot, so dass sofort erkannt werden kann, ob Körpertemperaturen von Tieren außerhalb vorgegebener Grenzen liegen.

Möglich ist es alternativ oder additiv auch, dass die Empfangsstation die Mittel zum akustischen Signalisieren eines außerhalb eines vorgegebenen Toleranzbereichs liegenden empfangenen Temperatursignals aufweist.

Die Zentralstation bzw. Datenverarbeitungseinheit kann zyklisch in regelmäßigen Abständen nacheinander alle Sende- und/oder Anzeigeeinheiten abfragen und die jeweiligen Temperaturen der individuellen Tiere protokollieren. Nach dem Eingang der individuellen Messwerte in der Zentralstation können diese visualisiert (z. B.: grün: zulässiger Temperaturbereich; rot: unzulässig hoher Temperaturbereich; blau: unzulässig niedriger Temperaturbereich) und gegebenenfalls ein Alarmhinweis ausgegeben werden, wenn unzulässige Temperaturdaten festgestellt wurden oder auch keine Messung erfolgte, die dann im Rahmen einer sich anschließenden tierärztlichen Diagnose beurteilt werden. Ausgegeben werden können bei Bedarf auch akustische Hinweise.

Als Folgemaßnahmen bei Feststellen einer Körpersaußentemperatur eines Tieres und/oder einer daraus ermittelten Körperinnentemperatur, die unzulässigerweise von einem Vergleichswert zu stark abweicht, kann das Tier - sofern es danach einen Laufweg, der eingezäunt ist, zurücklegen muss, durch automatisches umschalten einer Weiche separiert werden oder bei einem in einem Stand z.B. zumFressen oder Saufen stehenden Tier in diesem eingesperrt gehalten werden, bis weitere Folgemaßnahmen eingeleitet sind.

Folgemaßnahmen können das automatische Markieren des Tieres beispielsweise optisch mittels einem Farbcode sein, oder das insbesondere automatische, Ausstatten mit einem ortungsfähigen tag oder das sedieren mit einem dem Tier automatisch verabreichten Mittel, um weitere Folgemaßnahmen durchzuführen, insbesondere um das Tier nicht mehr in die freie Umgebung zu entlassen.

Ferner können neben der Temperatur weitere das Tier betreffende Daten dabei ermittelt werden, beispielsweise die Menge der Futteraufnahme oder Wasseraufnahme, die Distanzzeit zwischen den Besuchen an der Tierbehandlungsstelle, da solche Messungen durch Vergleich mit früheren analogen Daten desselben Tieres zusätzliche Hinweise auf eine Erkrankung bieten können, zu denen dann eine ungewöhnliche Temperatur des Tieres ein sekundäres Symptom wäre.

Der Erfindung löst somit insgesamt die Aufgabe, eine Vorrichtung, einen Bausatz und ein Verfahren jeweils zur Ermittlung der Körpertemperatur eines Nutztieres sowie ein zugehöriges Verfahren zur Messung der Körpertemperatur zu schaffen, mit der bzw. mit dem es möglich wird, die anfangs geschilderten Nachteile zu vermeiden.

Unter "bzw." ist im Rahmen der Anmeldung soweit sich keine andere Bedeutung ergibt "und/oder" zu verstehen.

Nachfolgend werden Ausführungsbeispiele der Erfindung anhand der Figuren beschrieben, die zeigen:
**Figur 1** zeigt schematisch in Draufsicht wie sich ein Nutztier einer erfindungsgemäßen Vorrichtung nähert, die unter Nutzung einer Futterstelle als Tierversorgungseinrichtung ausgebildet ist.
**Figur 2** zeigt schematisch die Situation, wenn das Nutztier aus Figur 1 die Futterstelle erreicht hat.
**Figur 3** zeigt eine erfindungsgemäße Vorrichtung umfassend ein Aufsteckteil zum Aufstecken bzw. Anstecken an ein Smartphone.
**Figur 4** zeigt schematisch einen erfindungsgemäßen Bausatz.

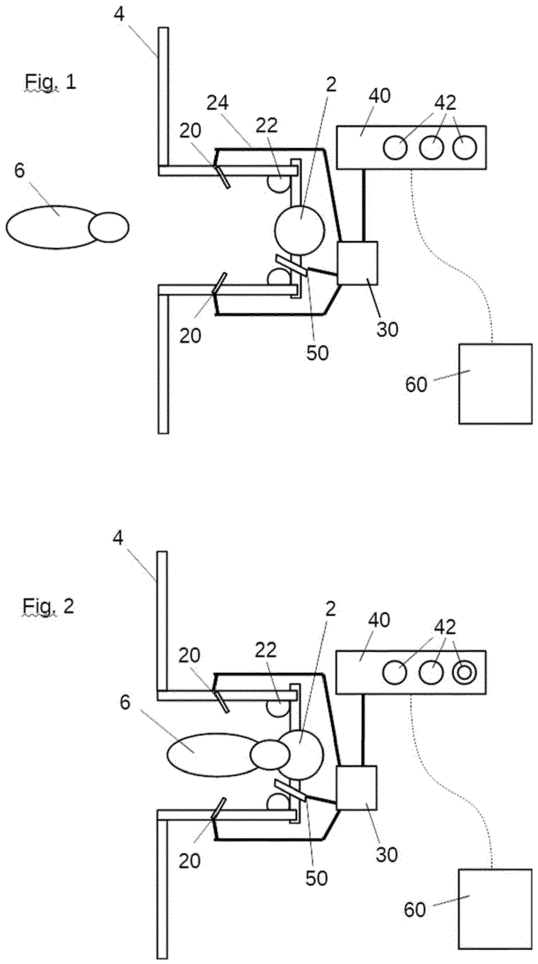

Wie in Figur 1 ersichtlich, kann eine erfindungsgemäße Vorrichtung basierend auf einer üblichen Futterstelle 2 als Tierversorgungseinrichtungen und/oder Tierpflegeeinrichtung der erfindungsgemäßen Vorrichtung ausgebildet werden. Entsprechende Futterstellen 2 sind üblicherweise wie auch im gezeigten Fall so ausgelegt, dass meist nur ein Nutztier 6 an die Futterstelle 2 gelangen kann. Dies erfolgt vorliegend mithilfe entsprechender Wandelemente 4 bzw. Gatterelemente 4 bzw. Zaunelemente 4.

Die erfindungsgemäße Vorrichtung umfasst ferner die Sensorelemente 20 bzw. Pyrometer 20 als Temperatursensor 20. Diese sind an den seitlich den Zugang zur Futterstelle 2 begrenzenden Wandungen 4 so angeordnet, dass sie an Punkten auf verschiedenen Seiten des Körpers eines die Futterstelle 2 nutzenden Nutztieres 6 die Oberflächentemperatur messen können.

Dabei sind die Pyrometer 20 am oberen Rand der Wandung 4 angeordnet, sodass sie im Allgemeinen von Nutztieren 6 nicht berührt werden können, und sind schräg nach unten gerichtet. Dadurch kann die Oberflächentemperatur der Nutztiere 6 erfasst werden, ohne dass die Nutztiere 6 durch Berührungen der Pyrometer 20 diese verschmutzen oder verschieben können.

Da die Pyrometer 20 schräg nach unten gerichtet sind, endet ihr Strahlweg bzw. ihr Blickwinkel jeweils in den unteren Ecken, die durch die die Futterstelle 2 haltende Wandung 4 und die seitlich begrenzenden Wandungen 4 ausgebildet sind.

Der untere Bereich dieser Ecken kann im Allgemeinen ebenfalls nicht durch die Nutztiere 6 erreicht werden, sodass hier Testflächen 22 angeordnet sind, deren Oberflächen-Temperatur die Pyrometer 20 messen können, solange kein Nutztier 6 die Futterstelle 2 aufsucht.

Ferner umfasst die erfindungsgemäße Vorrichtung einen RFID-Scanner 50, der so benachbart zu der Futterstelle 2 angeordnet ist, dass er einen im Ohr eines Nutztieres 6 implantierten RFID-Chip scannen bzw. lesen kann, wenn das Nutztier 6 aus der Futterstelle 2 frisst.

Pyrometer 20 und RFID-Scanner 50 sind über Kabel 24 mit der Auswerteeinheit 30 verbunden. Diese ist ausgelegt, die entsprechenden Sensordaten zu verarbeiten und beispielsweise bei einem Signal von dem RFID-Scanner 50, dass ein Nutztier identifiziert wurde und womit folglich das Nutztier 6 die Futterstelle 2 erreicht hat, eine Messung der Pyrometer 20 auszulösen.

Die Auswerteeinheit 30 ist über ein weiteres Kabel 24 mit der Sende- und/oder Anzeigeeinheit 40 verbunden und ferner dazu ausgelegt, diese entsprechend den gemessenen Oberflächen-Temperaturen, also sowohl der aktuell gemessenen als auch der von vergangenen Messungen bei demselben Nutztier 6, anzusteuern.

Die Sende- und/oder Anzeigeeinheit 40 umfasst im gezeigten Ausführungsbeispiel drei Leuchtmittel 42, die verschiedene Farbe aufweisen und so angeordnet sind, dass sie gut aus allen Richtungen und auch über die Wandung 4 hinweg sichtbar sind.

Beispielsweise könnte das in Figur 1 ganz rechts befindliche Leuchtmittel 42 rot sein und eine zu hohe Temperatur symbolisieren, das mittlere Leuchtmittel 42 könnte grün sein und eine in einem akzeptablen Normalbereich fallende Temperatur symbolisieren und das linke Leuchtmittel 42 könnte blau sein und eine zu niedrige Temperatur symbolisieren.

Die Sende- und/oder Anzeigeeinheit 40 steht ferner in drahtloser Verbindung mit der Datenverarbeitungseinheit als Zentralstation 60, beispielsweise über WLAN oder Bluetooth.

In der in Figur 1 gezeigten Situation hat sich zwar ein Nutztier 6 der Futterstelle 2 bereits genähert, jedoch noch nicht so weit, dass der RFID-Scanner 50 die Anwesenheit des RFID-Chip im Ohr bis Nutztieres 6 registrieren könnte, denn dieser hat nur eine sehr kurze Reichweite. Beispielsweise bezogen auf Figur 1 weniger als der Abstand RFID-Scanner 50 zum in Figur 1 unten befindlichen Pyrometer 20.

Nähert sich das Nutztiere 6 wie in Figur 2 gezeigt der Futterstelle 2, um zu fressen, kommt sein Kopf und damit sein entsprechendes Ohr dem RFID-Scanner 50 nah genug, dass dieser den im Ohr des Nutztiere 6 befindlichen RFID-Chip auslesen bzw. scannen kann.

Der RFID-Scanner 50 generiert ein Signal an die Auswerteeinheit 30, dass ein Nutztier 6 anwesend ist und welches Nutztier 6 dies ist bzw. dessen Identität.

Die Auswerteeinheit 30 löst daraufhin eine Messung durch die Pyrometer 20 aus.

Diese Messen die Oberflächen-Temperatur an Punkten auf gegenüberliegenden Seiten des Körpers des Nutztieres 6 und senden das jeweilige Messergebnis an die Auswerteeinheit 30.

Diese ist ausgelegt, die Messwerte der gegenüberliegenden Seiten auf Plausibilität numerisch zu überprüfen und daraus einen Wert für die tatsächliche Körpertemperatur des Nutztieres 6 zu bestimmen.

Die Auswerteeinheit 30 ist ferner dazu ausgelegt, die vorherigen Temperaturdaten dieses bestimmten Nutztieres 6 über die Sende- und/oder Anzeigeeinheit 40 aus der Datenverarbeitungseinheit bzw. Zentralstation 60 abzurufen und anhand eines Abgleichs abzuschätzen, ob der neuste Temperaturwert in einem akzeptablen Normbereich liegt oder außerhalb davon.

Ein solcher Abgleich ist insbesondere sinnvoll, da je nach individuellen Gesundheitszustandes des Nutztieres 6 und der entsprechenden Vorgeschichte möglicherweise eine leicht zu hohe oder zu niedrige Körpertemperatur ebenfalls in der konkreten Situation noch als akzeptabel einzuschätzen ist, beispielsweise, wenn sich das Nutztier 6 von einer Krankheit erholt und die Körpertemperatur zwar noch zu hoch ist aber bereits abfallend und ausreichend schnell abfällt.

Dieser Abgleich resultiere im gezeigten Fall der Figur 2 in dem Ergebnis, dass die Körpertemperatur des gezeigten Nutztieres 6 deutlich zu hoch und oberhalb des akzeptablen Normbereiches ist.

Entsprechend steuert die Auswerteeinheit 30 die Sende- und/oder Anzeigeeinheit 40 dazu an, dass das rechts gelegene rote Leuchtmittel 42 aufleuchtet.

Somit ist in der Anlage unmittelbar weithin sichtbar, dass das gerade an der Futterstelle 2 befindliche Nutztier 6 eine stark erhöhte Körpertemperatur hat. Damit können Nutzer bzw. Bedienpersonal der Anlage unmittelbar entsprechend reagieren, beispielsweise das Nutztier 6 von der restlichen Herde separieren und/oder einen Veterinärmediziner hinzuziehen.

Ferner steuert die Auswerteeinheit 30 die Sende- und/oder Anzeigeeinheit 40 dazu an, die gemessene Temperatur und die Identität des zugehörigen Nutztieres 6 an die Datenverarbeitungseinheit bzw. Zentralstation 60 zu übersenden.

Diese ist eingerichtet, eine Nutztierdatenbank zu enthalten, zu speichern und/oder zu pflegen. Entsprechend wird die Datenverarbeitungseinheit bzw. Zentralstation 60 den zu dem Nutztier 6 gehörenden Eintrag in der Nutztierdatenbank um die Messergebnisse/-daten der in der Figur 2 gezeigten Temperaturmessung zu ergänzen. Dabei kann in der Datenbank 60 sowohl der von der Auswerteeinheit 30 aus den gemessenen Oberflächen-Temperaturen bestimmte Wert für die Körpertemperatur gespeichert werden, als auch die unmittelbaren Messergebnisse der Pyrometer 20 selbst.

Dies ist vorteilhaft zum Identifizieren von Fehlern und gegebenenfalls zur Korrektur der in der Auswerteeinheit 30 genutzten Abschätzungsmethodik.

Wenn das Nutztiere 6 die Futterstelle verlassen hat und beispielsweise eine Situation ähnlich wie in Figur 1 gezeigt auftritt und sich kein weiteres Nutztier 6 bei der Futterstelle 2 befindet, bewirkt die Auswerteeinheit 30 eine weitere Temperaturmessung durch die Pyrometer 20. Diese messen aufgrund der Abwesenheit eines Nutztieres 6 nun die Oberflächen-Temperaturen der Testflächen 22.

Diese Messungen dienen der Kontrolle, dass die zuvor erfolgten Messungen der Temperatur nicht aufgrund einer Fehlfunktion eines oder beider Pyrometer 20 fehlerhaft sind.

Die Auswerteeinheit 30 ruft hierfür ferner aus der Datenverarbeitungseinheit bzw. Zentralstation 60 die Raumtemperaturen der letzten Stunden ab und kann so relativ präzise bestimmen, ob beide Pyrometer 20 in etwa die richtigen Oberflächen-Temperaturen messen.

Denn aufgrund der Anordnung der Testflächen 22 sind diese im Allgemeinen nicht verschmutzt und die Pyrometer 20 sind zu weit oberhalb der Tiere für eine Verschmutzung angeordnet, womit bei einwandfrei funktionierenden Pyrometern 20 die gemessene Oberflächen-Temperatur der Testflächen 22 genau der zu erwarteten Temperatur der Testflächen 22 aufgrund der Raumtemperatur entsprechen sollte.

Die Ergebnisse dieser Eichungsmessungen werden ebenfalls an die Datenverarbeitungseinrichtung bzw. Zentralstation 60 zur Einpflege in den Nutztierdatenbestand bzw. Datenbestand der Anlage übertragen.

Ferner könnte auch ein weiteres Leuchtmittel 42 an der Sende- und/oder Anzeigeeinheit 40 vorgesehen sein, das anzeigt, wenn die Kontrollmessung auf eine Fehlfunktion der Pyrometer 20 hindeutet.

Eine solche Kontrollmessung kann natürlich auch ständig erfolgen, sodass immer bei der in Figur 1 gezeigten oder ähnlichen Situationen Kontrollmessungen erfolgen. Alternativ zu ständigen Messungen oder Messungen, wenn das zuletzt vermessene Nutztiere 6 nicht mehr vom RFID-Scanner 50 erfasst werden kann, kann auch ein weiterer RFID-Scanner 50 im Zugangsbereich zur Futterstelle 2 vorgesehen sein, sodass eine Kontrollmessung der Oberflächen-Temperatur der Testflächen 22 erfolgt unmittelbar bevor sich das Nutztiere 6 der Futterstelle 2 soweit genähert hat, dass eine Messung der Oberflächentemperatur des Nutztieres 6 erfolgt.

Bevorzugt sind die Pyrometer 20 und/oder die Testflächen 22 und/oder der RFID-Scanner 50 so angeordnet, dass sie von dem im Bereich der Futterstelle 2 ohnehin üblicherweise erfolgenden regelmäßigen Reinigungsvorgang ebenfalls mit erfasst werden.

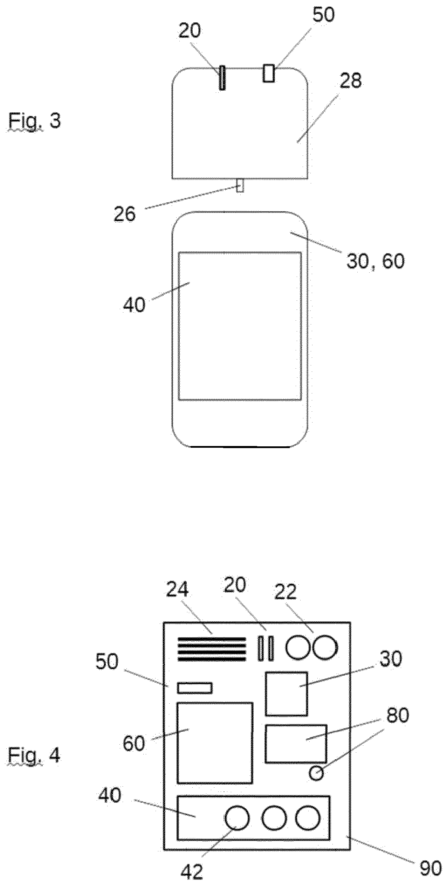

Figur 3 zeigt eine erfindungsgemäße Vorrichtung in der Ausführungsform mit Temperatursensor 20 als Aufsteckeinheit bzw. als Teil einer Aufsteckeinheit 28 zum Aufstecken und/oder Verbinden mit einem Smartphone.

Die Aufsteckeinheit 28 der gezeigten erfindungsgemäßen Vorrichtung umfasst dabei nicht nur den eigentlichen Temperatursensor bzw. das Pyrometer 20 sondern auch einen RFID-Scanner 50.

Diese sind entsprechend im Inneren der Aufsteckeinheit 28 mit dem Verbindungselement 26 verbunden, das zu einem entsprechenden Anschluss des Smartphones passt, beispielsweise einem USB-Anschluss.

Durch entsprechende Programmdaten oder Apps ist das Smartphone als Teil der erfindungsgemäßen Vorrichtung eingerichtet und Datenverarbeitungseinheit/- speicher, usw. des Smartphones dienen als Auswerteeinheit 30 und Zentralstation 60, während das Display des Smartphones als Sende-und/oder Anzeigeeinheit 40 dient.

Durch entsprechendes Aufstecken einer Aufsteckeinheit 28 und programmtechnische Einrichtung eines Smartphones lässt sich mit jedem gebräuchlichen Smartphone eine erfindungsgemäße Vorrichtung realisieren.

Dies ist insbesondere für Nutzer mit kleinen Herden an Nutztieren und auch für Besitzer von Haustieren nützlich, da sich mit relativ einfachen Mitteln und einem gewöhnlichen Smartphone eine erfindungsgemäße Vorrichtung ausbilden lässt.

Ein entsprechender Nutzer muss dann nur noch sein auf diese Weise eingerichtetes Smartphone mit dem Temperatursensor 20 und ggf. dem RFID-Scanner 50 auf ein entsprechendes Tier bzw. Nutztier ausrichten und kann eine Temperaturmessung durchführen. Diese wird dann unmittelbar im Speicher des Smartphones ähnlich der zuvor hinsichtlich Figuren 1 und 2 geschilderten Weise in eine Datenbank eingepflegt, die die vorherigen Temperaturmessungen am selben Tier enthält.

Ferner kann auf dem Display 40 des Smartphones auch unmittelbar angezeigt werden, ob die gemessene Temperatur auf irgendein gesundheitliches Problem des Tieres hindeutet.

Für die Aufsteckeinheit 28 kommt aber gerade auch für die Anwendung bei kleinen Herden und im Haustierbereich ein Weglassen des RFID-Scanners 50 infrage. Die Identifikation erfolgt dann durch den Nutzer des Smartphones und Eingabe des entsprechenden Tiernamens bzw. Kennzeichens.

Figur 4 zeigt einen erfindungsgemäßen Bausatz. Hierbei sind alle Elemente der in der Figur 1 gezeigten erfindungsgemäßen Vorrichtung bis auf die Futterstelle 2 in einer Transportkiste 90 gelagert.

Dies sind die zuvor geschilderten Elemente Pyrometer 20, Testflächen 22, Kabel 24, RFID-Scanner 50, Auswerteeinheit 30, Zentralstation 60 und Sende- und/oder Anzeigeeinheit 40 mit Leuchtmitteln 42. Ferner umfasst der gezeigte erfindungsgemäße Bausatz Aufbau- und Einrichtungshilfsmittel 80, beispielsweise entsprechende Werkzeuge und entsprechende Datenträger mit entsprechenden Programmdaten.

Mittels des in Figur 4 gezeigten erfindungsgemäßen Bausatzes lässt sich an Futterstellen entsprechend der Figuren 1 und 2 und auch an vielen anderen Typen von Tierversorgungseinrichtungen oder Tierpflegeeinrichtungen eine erfindungsgemäße Vorrichtung realisieren.

Aufgrund der Möglichkeit der unkomplizierten und transportablen Bereitstellung eines erfindungsgemäßen Bausatzes wie beispielsweise in Figur 4 gezeigt mittels der Kiste 90 eignet sich ein erfindungsgemäßer Bausatz insbesondere gut zur Umrüstung einer bestehenden Anlage und zur Realisierung einer erfindungsgemäßen Vorrichtung bei einer bestehenden Anlage.

## Patentansprüche

1. **Vorrichtung** zur berührungslosen Ermittlung der Körpertemperatur eines Tieres insbesondere Nutztieres (6), wobei die Vorrichtung aufweist:
- eine Tierversorgungseinrichtung (2) und/oder Tierpflegeeinrichtung, die für ein Tier (6) selbstständig erreichbar ist;
- einen Temperatursensor (20) zur Messung der Oberflächen-Temperatur an mindestens einer Stelle des Körpers des Tieres (6), wobei der Temperatursensor (20) so an oder benachbart zu der Tierversorgungseinrichtung (2) und/oder Tierpflegeeinrichtung angeordnet ist oder positionierbar ist, dass der Temperatursensor (20) das die Einrichtung aufsuchende Tier (6) erfasst;
- eine Auswerteeinheit (30), die mit dem Temperatursensor (20) für einen Datenaustausch in Verbindung steht, und die die vom Temperatursensor (20) gemessene Oberflächen-Temperatur erfasst, wobei die Auswerteeinheit (30) dazu eingerichtet ist, Temperaturdaten einem bestimmten Tier zuzuordnen und zu speichern; und
- eine Sende- und/oder Anzeigeeinheit (40), die mit der Auswerteeinheit (30) für einen Datenaustausch in Verbindung steht, insbesondere durch Fernkommunikation.

2. Vorrichtung nach Anspruch 1, wobei
die Tierversorgungseinrichtung (2) und/oder Tierpflegeeinrichtung eine Einrichtung ist, die regelmäßig von dem Tier selbsttätig aufgesucht wird;
die Sende- und/oder Anzeigeeinheit (40) ausgebildet und/oder eingerichtet ist zum Anzeigen und/oder Versenden von Temperaturdaten und darauf basierenden Informationen in Bezug auf das Tier (6);
die Auswerteeinheit (30) mit dem Temperatursensor (20) durch Fernkommunikation und/oder drahtlos in Verbindung steht;
die Auswerteeinheit (30) als eine Einheit und/oder integral mit dem Temperatursensor (20) ausgebildet ist; und/oder
der Temperatursensor (20) ein Pyrometer (20), insbesondere ein Infrarot-Pyrometer, und/oder eine Wärmebildkamera und/oder ein Infrarotsensor ist.

3. Vorrichtung nach einem der vorhergehenden Ansprüche, wobei der Temperatursensor (20) mindestens zwei Sensorelemente (20) umfasst, die bei Anordnung/Positionierung des Temperatursensors (20) an oder benachbart zur Tierversorgungseinrichtung (2) und/oder Tierpflegeeinrichtung die Oberflächen-Temperatur an unterschiedlichen Stellen des Körpers des Tieres (6).

4. Vorrichtung nach einem der vorhergehenden Ansprüche, wobei der Temperatursensor (20) mindestens zwei Sensorelemente (20) umfasst, die bei Anordnung/Positionierung des Temperatursensors (20) an oder benachbart zur Tierversorgungseinrichtung (2) und/oder Tierpflegeeinrichtung die Oberflächen-Temperatur aus unterschiedlichen Richtungen bezogen auf den Körper des Tieres (6) messen.

5. Vorrichtung nach einem der vorhergehenden Ansprüche, wobei der Temperatursensor (20) mindestens zwei Sensorelemente (20) umfasst, wobei mindestens ein Sensorelement (20) eine oder mehrere Stellen des Körpers des Tieres erfasst, die für mindestens ein anderes Sensorelement (20) durch den Körper des Tieres (6) verdeckt sind.

6. Vorrichtung nach einem der vorhergehenden Ansprüche, wobei die Vorrichtung
Mittel zur Eingabe und/oder Auswahl einer Tieridentität durch einen Nutzer;
ein Tieridentifikationsmittel (50) umfasst; und/oder
die Auswerteeinheit (30) als Tieridentifikationsmittel (50) eingerichtet ist.

7. Vorrichtung nach Anspruch 6,
wobei das Tieridentifikationsmittel (50) und/oder die Auswerteeinheit (30) einen Scanner, einen Barcode-Scanner, einen RFID-Scanner (50), einen Ohrmarken-Scanner und/oder eine Kamera umfasst, wobei das Tieridentifikationsmittel und/oder die Auswerteeinheit (30) bevorzugt eingerichtet ist zur Gesichtserkennung.

8. Vorrichtung nach einem der vorhergehenden Ansprüche, wobei die Anzeigeeinheit (40) mehrere Leuchtmittel (42) bevorzugt zur Erzeugung unterschiedlicher Farben und/oder mindestens einen Lautsprecher zur Erzeugung unterschiedlicher Signaltöne umfasst, wobei den Leuchtmitteln (42) und/oder den Signaltönen jeweilige Temperaturbereiche einer gemessenen Oberflächen-Temperatur in eindeutiger Weise zugeordnet sind und/oder wobei jeweiligen Temperaturbereichen in eindeutiger Weise bestimmte Leuchtmittel, Farben und/oder Signaltöne zugeordnet sind.

9. Vorrichtung nach einem der vorhergehenden Ansprüche, wobei der Temperatursensor (20), die Auswerteeinheit (30), das Tieridentifikationsmittel (50) und/oder die Sende- und/oder Anzeigeeinheit (40) mit Kabeln (24) und/oder Steckern und/oder elektronisch und/oder per Fernkommunikation verbunden sind und/oder integral ausgebildet sind und/oder als eine Einheit ausgebildet sind, bevorzugt verbunden mit oder als Teil eines Handhabungsmittels, welches es einem Nutzer erlaubt, den Temperatursensor (20) an oder benachbart zu der Tierversorgungseinrichtung (2) und/oder Tierpflegeeinrichtung zu positionieren.

10. Vorrichtung nach einem der vorhergehenden Ansprüche, wobei der Temperatursensor (20) als Aufsteckeinheit und/oder Teil einer Aufsteckeinheit (28) ausgebildet ist.

11. Vorrichtung nach Anspruch 10, wobei die Aufsteckeinheit zum Aufstecken und/oder Verbinden mit einem Smartphone ausgelegt ist, wobei das Smartphone besonders bevorzugt eingerichtet ist als Auswerteeinheit (30) und/oder Sende- und/oder Anzeigeeinheit (40), wobei bevorzugt die Stromversorgung des Temperatursensors über die Verbindung mit dem Smartphone oder mittels Batterie/Akku erfolgt.

12. Vorrichtung nach einem der vorhergehenden Ansprüche, wobei der Temperatursensor (20) eingerichtet ist, auf Anstieg einer gemessenen Temperatur, Aktivierung der Tierversorgungseinrichtung (2) und/oder Tierpflegeeinrichtung oder auf Ansprechen eines mit dem Temperatursensor (20) verbundenen oder darin integrierten Bewegungssensors und/oder Mikrophons eine Messung der Oberflächen-Temperatur und/oder eine gemessene Oberflächen-Temperatur an die Auswerteeinheit zu senden.

13. Vorrichtung nach einem der vorhergehenden Ansprüche, wobei die Auswerteeinheit (30) dazu eingerichtet ist, basierend auf Oberflächen-Temperaturen eines bestimmten Tieres (6) Folgemaßnahmen auszulösen, wie beispielsweise Aktivierung oder angepasste Aktivierung der Tierversorgungseinrichtung (2) und/oder Tierpflegeeinrichtung und/oder Warnsignalen, insbesondere optischen oder akustischen oder elektronischen, und/oder Änderung/Markierung eines Datenbankeintrags des bestimmten Tieres und/oder Benachrichtigungen, insbesondere elektronischen, und/oder Verabreichung von besonderen Tierpflegemitteln wie Medikamenten und/oder Umleitung des Tieres (6) in einen speziellen Pflegebereich.

14. Vorrichtung nach einem der vorhergehenden Ansprüche ferner umfassend eine Datenverarbeitungseinheit (60) zur Speicherung und Pflege eines Tierdatenbestandes, bevorzugt als Zentralstation (60), wobei die Sende- und/oder Anzeigeeinheit (40) ferner eingerichtet ist, Temperaturdaten mit Referenz auf das zugehörige bestimmte Tier (6) an die Datenverarbeitungseinheit (60) zu senden, wobei die Datenverarbeitungseinheit (60) dazu eingerichtet ist, Befehlssignale an die Tierversorgungseinrichtung (2) und/oder Tierpflegeeinrichtung, die Auswerteeinheit (30) und/oder die Sende- und Anzeigeeinheit (40) zu senden, um anhand des Tierdatenbestandes als erforderlich ermittelte Vorgänge auszulösen.

15. Vorrichtung nach einem der vorhergehenden Ansprüche, wobei die Auswerteeinheit (30) ferner dazu eingerichtet ist, in Abwesenheit eines Tieres (6) Messungen von Neutraltemperaturen insbesondere einer Testfläche (22) auszuführen, und bei ungewöhnlichen Neutraltemperaturen, insbesondere in Anbetracht einer Raumtemperatur, welche beispielsweise an die Auswerteeinheit (30) regelmäßig von einem Raumtemperatursensor übermittelt wird, ein Störungssignal auszulösen oder zu übertragen, insbesondere an die Sende- und Anzeigeeinheit (40).

16. Vorrichtung nach einem der vorhergehenden Ansprüche, wobei der Temperatursensor (20) und/oder andere oder zusätzliche Elemente der Vorrichtung dazu eingerichtet ist, eine Entfernung zwischen Temperatursensor (20) und der mindestens einen Stelle des Körpers des Tieres (6) zu erfassen bzw. zu ermitteln, wobei bevorzugt die Auswerteeinheit (30) und/oder die Datenverarbeitungseinheit (60) ferner dafür ausgelegt ist, die Entfernung zu speichern, zu katalogisieren und/oder eine korrigierte Temperatur unter Verwendung von wenigstens der gemessenen Oberflächen-Temperatur und der Entfernung zu bestimmen.

17. Bausatz und/oder Baukasten zur Ausbildung einer Vorrichtung zur berührungslosen Ermittlung der Körpertemperatur eines Tieres, wobei der Bausatz und/oder Baukasten aufweist:
- mindestens einen Temperatursensor (20) zur Messung der Oberflächen-Temperatur an mindestens einer Stelle des Körpers des Tieres;
- eine Verbindungseinrichtung zum Verbinden oder benachbart Anordnen des Temperatursensors mit bzw. zu einer Tierversorgungseinrichtung (2) und/oder Tierpflegeeinrichtung;
- eine Auswerteeinheit (30), die mit dem Temperatursensor (20) für einen Datenaustausch verbindbar ist, insbesondere durch Fernkommunikation, und die zum Erfassen der von dem Temperatursensor (20) gemessenen Oberflächen-Temperatur ausgelegt ist, und/oder einen Datenträger (80), der mit Programmdaten eingerichtet ist zur Einrichtung von einer im Umfeld der Tierversorgungseinrichtung (2) und/oder Tierpflegeeinrichtung vorhandenen Datenverarbeitungseinheit als Teil der oder insgesamt als Auswerteeinheit (30); und
- eine Sende- und/oder Anzeigeeinheit (40), die mit der Auswerteeinheit (30) für einen Datenaustausch verbindbar ist oder integral mit dieser ausgebildet ist und/oder den bzw. einen Datenträger (80), der mit Programmdaten eingerichtet ist zur Einrichtung von einem im Umfeld der Tierversorgungseinrichtung (2) und/oder Tierpflegeeinrichtung vorhandenen Datenverarbeitungseinheit als Teil der oder insgesamt als Sende- und/oder Anzeigeeinheit,
wobei der Bausatz und/oder Baukasten ausgelegt ist, durch Anordnung und/oder Installation an oder bei einer Tierversorgungseinrichtung (2) und/oder Tierpflegeeinrichtung eine Vorrichtung nach einem der vorhergehenden Ansprüche auszubilden.

18. **Verfahren,** bevorzugt bei Nutzung einer Vorrichtung nach einem der Ansprüche 1 bis 16 oder einer durch einen Bausatz/Baukasten nach Anspruch 17 ausgebildeten Vorrichtung, umfassend die Schritte:
- berührungsloses Messen der Oberflächen-Temperatur eines Tieres insbesondere Nutztieres (6) bevorzugt mittels der Vorrichtung;
- Identifizieren des Tieres (6);
- Vergleich der gemessenen Oberflächen-Temperatur mit früher gemessenen Oberflächen-Temperaturen desselben Tieres, insbesondere an der gleichen Stelle des Körpers des Tieres (6);
- Auslösung einer Folgefunktion abhängig von dem Ergebnis des, Vergleichs; und
- Speicherung der gemessenen Oberflächen-Temperatur des Tieres (6), bevorzugt in Verbindung mit anderen Daten wie Uhrzeit, Datum, Fütterungsmenge, Verweildauer des Tieres in einer Tierversorgungseinrichtung und/oder Tierpflegeeinrichtung, Menge der gemolkenen Milch;
wobei alle Schritte mindestens einmal pro Woche, bevorzugt mindestens einmal pro 24 Stunden, ausgeführt werden, wobei das Verfahren bevorzugt ferner den Schritt umfasst:
Messen der Oberflächen-Temperatur einer Testfläche (22), bevorzugt zur Kalibrierung eines Temperatursensors.
